(19) Europäisches Patentamt
European Patent Office
Office européen des brevets

(11) Veröffentlichungsnummer: **0 086 320**
**B1**

(12) **EUROPÄISCHE PATENTSCHRIFT**

(45) Veröffentlichungstag der Patentschrift: **13.11.85**

(51) Int. Cl.⁴: **C 07 C 103/38**, A 61 K 31/16

(21) Anmeldenummer: **83100001.3**

(22) Anmeldetag: **03.01.83**

(54) **Panthenolderivate.**

(30) Priorität: **12.02.82 CH 890/82**

(43) Veröffentlichungstag der Anmeldung: **24.08.83 Patentblatt 83/34**

(45) Bekanntmachung des Hinweises auf die Patenterteilung: **13.11.85 Patentblatt 85/46**

(84) Benannte Vertragsstaaten: **AT BE CH DE FR GB IT LI LU NL SE**

(56) Entgegenhaltungen: **DE - C - 840 839**

(73) Patentinhaber: **F. HOFFMANN-LA ROCHE & CO. Aktiengesellschaft, CH-4002 Basel (CH)**

(72) Erfinder: **Hanck, Alfred, Dr., Kilchgrundstrasse 10, CH-4125 Riehen (CH)**
Erfinder: **Pauling, Horst, Dr., Ruchholzstrasse 39, CH-4103 Bottmingen (CH)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

EP 0 086 320 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Panthenolderivate, insbesondere betrifft sie neue Salze von Panthenol, und zwar Aluminium-D oder DL-panthenolat und Zink-D oder DL-panthenolat.

Die neuen Verbindungen entsprechen der allgemeinen Formel

D oder DL

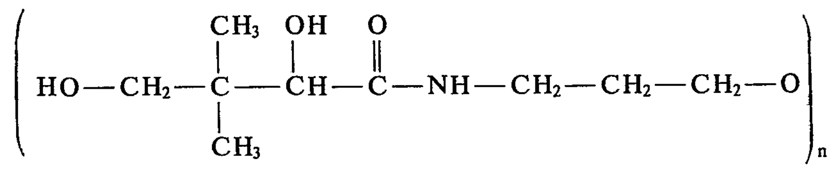

(I)

worin Me Aluminium und n die Zahl 3, oder Me Zink und n die Zahl 2 bedeuten.

Die Erfindung betrifft ferner die Herstellung der Verbindungen der Formel I sowie deren Verwendung als topische Arzneimittel.

Aus Römpps Chemie Lexikon, 7. Auflage, Seite 2492 und 8. Auflage, Seite 571, ist die Verwendung von Panthenol sowie von Natrium- und Calciumsalzen der Pantothensäure in Dermatika bekannt. Weiterhin sind aus Römpps Chemie-Lexikon, 7. Auflage, Seiten 3990 bis 3991 und 8. Auflage, Seite 157, Zink- und Aluminiumpräparate mit antiseptischen Eigenschaften bekannt.

Die Verbindungen der Formel I können dadurch hergestellt werden, daß man D- oder DL-Panthenol der Formel

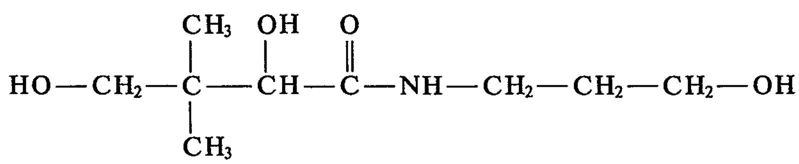

(II)

mit einer Verbindung der allgemeinen Formeln

$(R)_3Al$ (III) oder $(R)_2AlH$ (IV)

worin R $C_1-C_{12}$-Alkyl- oder $C_1-C_{12}$-Alkoxygruppen darstellt

im Molverhältnis 3 : 1 oder mit einer Verbindung der allgemeinen Formel

$(R)_2Zn$ (V)

worin R die obige Bedeutung hat

im Molverhältnis 2 : 1 umsetzt.

Der Ausdruck $C_1-C_{12}$-Alkyl bedeutet im Rahmen der vorliegenden Erfindung sowohl geradkettige als auch verzweigte Alkylgruppen mit 1 bis 12 Kohlenstoffatomen, vorzugsweise solche mit 2 bis 8 Kohlenstoffatomen, wie z. B. Aethyl, Propyl, Isopropyl, n-Butyl, Isobutyl, tert. Butyl, Pentyl, Hexyl usw. Eine besonders bevorzugte Alkyl-Aluminiumverbindung ist das Triisobutylaluminium und eine besonders bevorzugte Alkylzinkverbindung ist das Diäthylzink.

Der Ausdruck $C_1-C_{12}$-Alkoxy bedeutet im Rahmen der vorliegenden Erfindung Alkoxygruppen, in denen der Alkylrest obige Bedeutung hat. Es handelt sich bei den Verbindungen der Formeln III—V, worin R Alkoxy bedeutet, um Alkoholate, wobei diejenigen bevorzugt sind, welche Alkoholen abgeleitet sind, welche einen Siedepunkt unterhalb 118—120° C bei 1,33 Pa aufweisen. Bevorzugte Alkoholate sind Aluminium- und Zinkisopropylat.

Die Umsetzung von D- oder DL-Panthenol mit einer Verbindung der Formeln III—V, worin R Alkyl darstellt, kann zweckmäßig in einem aprotischen wasserfreien Lösungsmittel sowie unter einem trockenen Schutzgas, wie Stickstoff oder Argon, durchgeführt werden. Geeignete Lösungsmittel sind z. B. aliphatische Kohlenwasserstoffe, wie Hexan oder Heptan, aromatische Kohlenwasserstoffe, wie Benzol oder Toluol, chlorierte Kohlenwasserstoffe, wie Methylenchlorid oder Chlorbenzol, sowie Aether, wie Diäthyläther oder Anisol und dergleichen. Die Umsetzung verläuft im allgemeinen rasch und quantitativ. Da sich die bildenden Kohlenwasserstoffe und der Wasserstoff entweder Gase oder tiefsiedende Flüssigkeiten sind, können diese leicht abgetrennt werden. Bei Verwendung reiner Ausgangsmaterialien gelangt man in einem Schritt sofort zu analysenreinen Produkten, die keiner weiteren Reinigung mehr bedürfen.

Zur Herstellung von Aluminium-D-panthenolat wird vorzugsweise D-Panthenol mit Triisobutylaluminium umgesetzt. Zur Herstellung der D,L-Form geht man von D,L-Panthenol aus. Anstelle von Triisobutylaluminium kann auch eine Bis-niederalkyl-aluminiumhydrid-Verbindung, vorzugsweise Diisobutylaluminiumhydrid verwendet werden.

Zur Herstellung von Zink-D-Panthenolat wird vorzugsweise D-Panthenol mit Diäthylzink umgesetzt. Zur Herstellung der D,L-Form geht man von D,L-Panthenol aus.

Bei der Umsetzung von D- oder D,L-Panthenol mit einer Verbindung der Formeln III—V, worin R Alkoxy darstellt, handelt es sich um eine Umsetzung eines Alkoholates des Aluminium bzw. des Zink mit dem Panthenol. Hierbei wird der Alkohol ROH durch das Panthenol verdrängt, was besonders gut gelingt, wenn der zu verdrängende Alkohol niedriger siedet als Panthenol. Bei diesem Verfahren können die vorhergehend erwähnten Lösungsmittel ebenfalls verwendet werden, wobei es jedoch noch von Vorteil ist, in einem Lösungsmittel zu arbeiten, das mit dem aus dem Reaktionsgemisch zu enfernenden Alkohol ROH ein Azeotrop bildet. Unter Verwendung reiner Ausgangsmaterialien erhält man auf diese Weise nach Abtrennen des Alkohols ROH und des Lösungsmittels in einem Reaktionsschritt sofort analysenreine Endprodukte.

Zur Herstellung von Aluminium-D-Panthenolat wird vorzugsweise D-Panthenol mit Aluminiumisopropylat umgesetzt. Zur Herstellung von Zink-D-Panthenolat wird vorzugsweise D-Panthenol mit Zinkisopropylat umgesetzt. Zur Herstellung der D,L-Formen geht man von D,L-Panthenol aus.

Die für das erfindungsgemäße Verfahren verwendeten Ausgangsmaterialien sind entweder bekannt und z. T. in genügender Reinheit käuflich oder können in analoger Weise zu den bekannten Verbindungen hergestellt werden.

Die erfindungsgemäßen Verbindungen der Formel I, die sich an der Maus als sehr wenig toxisch erwiesen haben, kommen als topische Arzneimittel in Betracht. Die Verbindungen der Formel I haben sich am Meerschweinchen als praktisch nicht hautreizend und als nicht allergisch erwiesen, was für ein topisches Arzneimittel außerordentlich günstig ist.

Die erfindungsgemäßen topischen Arzneimittel kommen insbesondere bei der Wundheilung in Betracht. Sie unterstützen die Wundheilung und Epithelisierung wie z. B. bei Brand-, Schürf- und Schnittwunden, bei chronischen Ulzera, Dekubitus und Analfissuren und dienen zur Nachbehandlung bei Hauttransplantationen und operativen Eingriffen bei Zervixerosionen. Die erfindungsgemäßen Verbindungen eignen sich ebenfalls zur Prophylaxe und Therapie von Brustrhagaden, sowie Windelerythemen und Sonnenbrand. Weiterhin hat sich gezeigt, daß die Reißfestigkeit von Operationsnarben bei Verwendung der erfindungsgemäßen Verbindungen signifikant zunimmt. Ganz allgemein wird bei der Verwendung der erfindungsgemäßen Verbindungen eine schnellere und bessere Wundheilung erzielt. Es hat sich auch schon herausgestellt, daß die erfindungsgemäßen Verbindungen der Formel I eine Hemmwirkung auf das Wachstum von Eitererregern besitzen, also eine antiseptische Wirkung haben und überraschenderweise gleichzeitig eine Aktivierung der Makrophagen bewirken. Erfindungsgemäß können die D- oder DL-Formen eingesetzt werden, doch sind die D-Isomeren bevorzugt.

Erfindungsgemäß werden die Verbindungen der allgemeinen Formel I topisch auf das Hautgewebe gebracht. Die Verbindungen der allgemeinen Formel I werden zweckmäßigerweise in Form von pharmazeutischen Präparaten verwendet, welche eine wirksame Menge einer Verbindung der Formel I und ein pharmazeutisch annehmbares, für die topische Anwendung geeignetes Trägermaterial enthalten. Erfindungsgemäße topische Dosierungsformen enthalten, bezogen auf ihr Gesamtgewicht, in der Regel etwa 0,1 bis 10 Gewichtsprozent, vorzugsweise 0,5 bis 5 Gewichtsprozent, einer Verbindung der allgemeinen Formel I. Je nach Dosierungsform können jedoch auch höhere oder niedere Konzentrationen vorliegen.

Der Ausdruck »topisch«, wie er in der vorliegenden Beschreibung verwendet wird, bezieht sich auf die Verwendung des aktiven Bestandteils, das mit einem geeigneten Trägermaterial verarbeitet und auf die Haut oder Schleimhaut gebracht wird, so daß es die lokale Wirkung entfalten kann. Demgemäß umfassen die topischen Arzneimittel für die externe Anwendung geeignete, pharmazeutische Dosierungsformen, so daß ein direkter Kontakt mit der Haut entsteht. Die topischen Dosierungsformen umfassen Gele, Crèmes, Lotionen, Salben, Puder, Aerosole und andere herkömmliche Formen, welche für die direkte Applikation von Arzneimitteln auf die Haut oder Schleimhaut geeignet sind. Diese Dosierungsformen können hergestellt werden, indem man Verbindungen der allgemeinen Formel I mit bekannten pharmazeutischen, für die topische Anwendung geeigneten Trägermaterialien vermischt.

Salben und Crèmes enthalten ölige, absorbierende, wasserlösliche und/oder emulgierende Trägermaterialien, wie Vaseline, Paraffinöl, Propylenglykol, Cetylalkohol, Glycerinmonostearat oder alkylverzweigte Fettsäuren und dergleichen.

Lotionen sind flüssige Präparate und können von einfachen Lösungen bis zu wäßrigen oder wäßrig/alkoholischen Präparaten, welche die Substanzen in fein verteilter Form enthalten, variieren. Die Präparate enthalten suspendierende oder dispergierende Stoffe, wie z. B. Natriumcarboxymethylcellulose, welche den Wirkstoff in einem aus Wasser, Alkohol, Glycerin und dergleichen hergestellten Träger suspendieren bzw. dispergieren.

Gele sind halbfeste Präparate, welche man durch Gelieren einer Lösung oder Suspension des Wirkstoffes in einem Trägermaterial erhält. Die Trägermaterialien, welche hydrophil oder hydrophob sein können, werden unter Verwendung eines Geliermittels, wie Carbopol und dergleichen, geliert.

Aerosole sind Lösungen oder Suspensionen des Wirkstoffes in einem inerten Trägermaterial, welche unter Verwendung von speziellen Sprüheinrichtungen verteilt werden. Üblicherweise verwendete Träger sind Trichlormonofluormethan und Trichlordifluormethan.

Die nachfolgenden Beispiele erläutern die Erfindung.

## Beispiel 1

In einem 1-Liter-Vierhalskolben, versehen mit einem kräftigen Rührer, 250-ml-Tropftrichter mit Druckausgleich und Rückflußkühler, sowei einer Vorrichtung zum Arbeiten unter Schutzgas, werden unter Inertgas bei Raumtemperatur 72,8 g D-Panthenol (354,7 mMol) und 400 ml absolutes Dichlormethan gegeben. Da sich das D-Panthenol schlecht in Dichlormethan löst, wird kräftig gerührt und auf 5° C im Eisbad abgekühlt. Zu dieser Emulsion werden innert einer Stunde 23,46 g Triisobutylaluminium (30,0 ml, 118,2 mMol), gelöst in 150 ml absolutem Toluol, zugetropft. Man erhält dabei eine weiße, rührbare Masse. Wenn alles zugetropft ist, läßt man auf Raumtemperatur und danach auf 60°C (Badtemperatur) erwärmen und destilliert das Dichlormethan ab. Dann wird während zwei Stunden bei der Rückflußtemperatur des Toluols erhitzt. Nach Abkühlen auf ca. 50° C wird das Toluol zuerst an der Wasserstrahlpumpe entfernt, worauf man bis zur Gewichtskonstanz an der Ölpumpe trocknet. Man trocknet 24 Stunden bei 50°C im Vakuumtrockenschrank an der Ölpumpe, wobei das Produkt dabei allmählich fest wird. Nach dem Erkalten läßt sich die farblose amorphe Masse im Mörser zu einem weißen Pulver zerkleinern, das leicht hygroskopisch ist. Man erhält 73,4 g Aluminium-D-Panthenolat vom Schmelzintervall 78—82° C und einer spezifischen Drehung: $[\alpha]_D^{20}$ = +47,8° C (c = 1 in Dimethylsulfoxid).

## Beispiel 2

a) In einen 500-ml-3-Halskolben, versehen mit Tropftrichter, Rührer und Vorrichtung zum Arbeiten unter Schutzgas werden unter Schutzgas 9,1 ml absolutes Isopropanol (= 7,1 g ≙ 118,3 mMol), sowie 300 ml absolutes n-Hexan gegeben und auf ca. 5° C abgekühlt. Unter gutem Rühren wird eine Lösung von 10 ml Triisobutylaluminium (= 7,82 g ≙ 39,4 mMol) in 60 ml absolutem Hexan zugetropft. Danach läßt man durch Entfernen des Kühlbades die Temperatur im Reaktionskolben auf Raumtemperatur kommen und erwärmt danach für zwei Stunden auf 50°C. Man erhält auf diese Weise eine klare Lösung von Aluminiumisopropylat in n-Hexan.

b) 24,3 g D-Panthenol (= 118,3 mMol) werden in eine gleiche, wie oben unter a) beschriebene Apparatur vorgelegt. Dazu wird die gemäß a) hergestellte Aluminiumisopropylatlösung in n-Hexan gegeben. Man rührt zwei Stunden bei Raumtemperatur und erhitzt vier Stunden unter Rückfluß. Danach destilliert man n-Hexan und Isopropanol bei Normaldruck ab. Aus dem Tropftrichter wird solange absolutes n-Hexan (ca. 300 ml) zugegeben, bis im Destillat gaschromatographisch kein Isopropanol mehr nachweisbar ist. Danach wird das restliche Hexan zuerst unter Normaldruck, zuletzt (bei ca. 65°C Heizbadtemperatur) im Ölpumpenvakuum, während zwei Stunden abgezogen, wobei man 25,2 g Aluminium-D-Panthenolat erhält.

## Beispiel 3

In einen 1-Liter-Vierhalskolben, versehen mit Rührer, 100-ml-Tropftrichter mit Druckausgleich und Rückflußkühler sowie einer Vorrichtung zum Arbeiten unter Schutzgas, werden unter Inertgas bei Raumtemperatur 58,90 g D-Panthenol (287 mMol) und 600 ml n-Hexan (reinst) gegeben und kräftig gerührt. Das D-Panthenol ist in n-Hexan schwer löslich. Wenn das D-Panthenol zu kleinen Tröpfchen emulgiert ist, werden aus dem Tropftrichter 17,73 g Diäthylzink (15,0 ml = 143,5 mMol), verdünnt in 80 ml n-Hexan (reinst), innert einer Stunde zugetropft. Die Reaktion beginnt sofort, erkennbar am Entweichen von Aethan; der Ansatz erwärmt sich schwach. Nach ca. einem Drittel der Zugabe der Diäthylzinklösung erhält man im Reaktionskolben eine zähe, schwer rührbare Masse. Es wird nun im Eisbad auf 5° C abgekühlt, dabei wird die Masse fest und zu einem feinen Pulver zerkleinert. Man läßt das Reaktionsgemisch langsam auf Raumtemperatur kommen und setzt das Zutropfen fort. Wenn alles zugetropft ist, wird auf Rückflußtemperatur erwärmt und eine Stunde bei dieser Temperatur gerührt, danach läßt man wieder auf Raumtemperatur abkühlen und rührt über Nacht. Danach werden 250 ml n-Hexan abdestilliert, dann wird wieder auf 5° C abgekühlt (im Eisbad), worauf man das Reaktionsgemisch zwei Stunden ohne Rühren stehen läßt. Das Produkt setzt sich ab, und das n-Hexan läßt sich gut abdekantieren. Der Rest des n-Hexans wird an der Wasserstrahlpumpe abgezogen und das Produkt an der Ölpumpe bis zur Gewichtskonstanz getrocknet. Da das Produkt hygroskopisch ist, wird es unter Feuchtigkeitsausschluß aufbewahrt. Man erhält 60,73 g Zink-D-Panthenolat als weißes amorphes Pulver mit einem Schmelzintervall von 105—107°C und einer spezifischen Drehung von $[\alpha]_D^{20}$ = +31° C (c = 1% in Dimethylsulfoxid).

## Beispiel 4

a) In einem 500-ml-Vierhalskolben, versehen mit Rührer, Thermometer, Tropftrichter, sowie einem Rückflußkühler mit einer Vorrichtung zum Arbeiten unter Schutzgas, wird unter Argon eine Lösung von 11,5 g absolutem Isopropanol (14,75 ml ≙ 191,4 mMol) in 300 ml absolutem n-Hexan vorgelegt und auf 5° C abgekühlt. Dazu tropft man langsam eine Lösung von 10 ml Diäthylzink (11,82 g ≙ 95,7 mMol) in 60 ml absolutem n-Hexan. Nach beendeter Zugabe wird zwei Stunden auf 50° C erwärmt.

b) Zu der oben hergestellten Suspension von Zinkisopropylat in n-Hexan werden bei Raumtemperatur 39,3 g D-Panthenol (191,4 mMol) gegeben. Unter Rühren werden bei Normaldruck n-Hexan und Isopropanol abdestilliert, wobei frisches n-Hexan solange zugegeben wird (ca. 300 ml), bis sich gaschromatographisch im Destillat kein Isopropanol mehr nachweisen läßt. Danach wird das restliche Lösungsmittel zuerst im Wasserstrahlpumpenvakuum, dann während zwei Stunden im Ölpumpenvakuum, bis zur Gewichtskonstanz getrocknet, wobei man 42,9 g Zink-D-Panthenolat erhält.

## Beispiel A

Wundgel in neutraler Gelgrundlage mit folgender Zusammensetzung wird in an sich bekannter Weise hergestellt:

| | |
|---|---|
| Zink-D-Panthenolat | 5,0 g |
| Glycofurol | 9,0 g |
| Isopropylalkohol | 11,0 g |
| Neo-PCL | 68,0 g |
| Aerosil 200 | 7,0 g |

## Beispiel B

Wundgel in neutraler Gelgrundlage mit folgender Zusammensetzung wird in an sich bekannter Weise hergestellt:

| | |
|---|---|
| Aluminium-D-Panthenolat | 5,0 g |
| Glycofurol | 9,0 g |
| Isopropylalkohol | 12,0 g |
| Neo-PCL | 68,0 g |
| Aerosil 200 | 6,0 g |

## Beispiel C

Salbe der Zusammensetzung:

| | |
|---|---|
| Aluminium-D-Panthenolat | 1,0 g |
| Glycofurol | 9,0 g |
| Miglyol-Gel | 90,0 g |

### Herstellung eines repräsentativen Ansatzes

1 g Aluminium-D-Panthenolat wird in 9,0 g Glycofurol gelöst und mit 10,0 g Miglyol-Gel gemischt. (Miglyol-Gel ist ein pharmazeutischer Hilfsstoff, bestehend aus einem Triglycerid-Gemisch gesättigter Pflanzenfettsäuren.)

## Beispiel D

Gel der Zusammensetzung:

| | |
|---|---|
| Aluminium-D-Panthenolat | 1,0 g |
| Glycofurol | 9,0 g |
| Isopropylalkohol | 12,0 g |
| Neo-PCL wasserlöslich | 72,0 g |
| Aerosil 200 | 6,0 g |

Herstellung eines repräsentativen Ansatzes

1 g Aluminium-D-Panthenolat wird in 9,0 g Glycofurol gelöst und mit 12,0 g Isopropylalkohol vermischt. Diese Mischung wird zu 72,0 g Neo-PCL wasserlöslich gegeben und zum Schluß werden 6,0 g Aerosil 200 eingerührt. (Neo-PCL wasserlöslich ist ein Markenpräparat der Firma Dragoco und besteht aus einem Gemisch alkylverzweigter Fettsäuren. Aerosil 200 ist Siliziumdioxid.)

Beispiel E

Puder der Zusammensetzung:

| | |
|---|---|
| Aluminium-D-Panthenolat | 25,0 g |
| Magnesiumstearat | 2,36 g |
| Talk | 64,75 g |
| Zinkoxid | 7,89 g |

Herstellung eines repräsentativen Ansatzes

25,0 g Aluminium-D-Panthenolat werden zusammen mit 2,36 g Magnesiumstearat, 64,75 g Talk und 7,89 g Zinkoxid durch ein Sieb (0,5 mm) gesiebt und in einem Mischer während 15 Minuten gemischt.

Beispiel F

Puder der Zusammensetzung:

| | |
|---|---|
| Zink-D-Panthenolat | 5,0 g |
| Magnesiumstearat | 3,0 g |
| Talk | 82,0 g |
| Zinkoxid | 10,0 g |

Herstellung eines repräsentativen Ansatzes

5 g Zink-D-Panthenolat werden zusammen mit 3,0 g Magnesiumstearat, 82 g Talk und 10 g Zinkoxid durch ein Sieb (0,5 mm) gesiebt und in einem Mischer während 15 Minuten gemischt.

**Patentansprüche für die Vertragsstaaten BE, CH, DE, FR, GB, IT, LI, LU, NL, SE**

1. Verbindungen der allgemeinen Formel

D oder DL

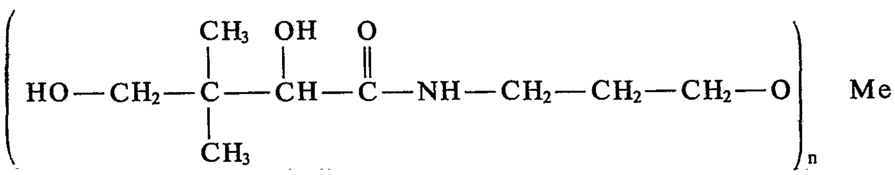

(I)

worin Me Aluminium und n die Zahl 3, oder Me Zink und n die Zahl 2 bedeuten.

2. Aluminium-D-panthenolat.
3. Zink-D-panthenolat.
4. Eine Verbindung gemäß Anspruch 1, 2 oder 3 als topisches Arzneimittel.
5. Eine Verbindung gemäß Anspruch 1, 2 oder 3 als topisches Wundheilmittel.
6. Verfahren zur Herstellung von Verbindungen der allgemeinen Formel I, gemäß Anspruch 1, dadurch gekennzeichnet, daß man D- oder DL-Panthenol der Formel

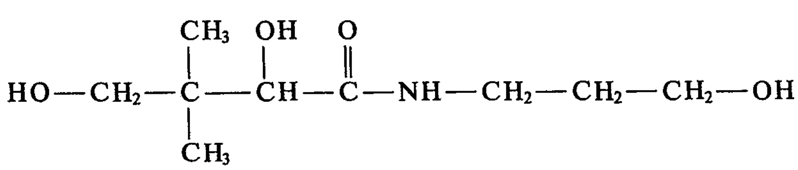

(II)

mit einer Verbindung der allgemeinen Formeln

(R)$_3$Al (III) oder (R)$_2$AlH (IV)

worin R $C_1-C_{12}$-Alkyl- oder $C_1-C_{12}$-Alkoxygruppen darstellt,

im Molverhältnis 3 : 1 oder mit einer Verbindung der allgemeinen Formel

(R)$_2$Zn (V)

worin R die obige Bedeutung hat,

im Molverhältnis 2 : 1 umsetzt.

7. Topisches Arzneimittel, enthaltend eine Verbindung der allgemeinen Formel I gemäß Anspruch 1.

8. Topisches Wundheilmittel, enthaltend einer Verbindung der Formel I gemäß Anspruch 1.

**Patentansprüche für den Vertragsstaat AT**

1. Verfahren zur Herstellung von Verbindungen der allgemeinen Formel

D oder DL

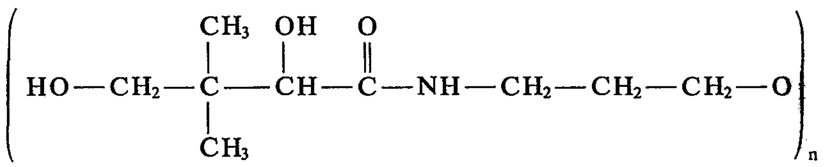

(I)

worin Me Aluminium und n die Zahl 3, oder Me Zink und n die Zahl 2 bedeuten,

dadurch gekennzeichnet, daß man D- oder DL-Panthenol der Formel

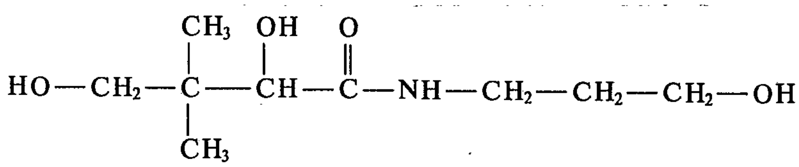

(II)

mit einer Verbindung der allgemeinen Formeln

(R)$_3$Al (III) oder(R)$_2$AlH (IV)

worin R $C_1-C_{12}$-Alkyl- oder $C_1-C_{12}$-Alkoxygruppen darstellt,

im Molverhältnis 3 : 1 oder mit einer Verbindung der allgemeinen Formel

(R)$_2$Zn (V)

worin R die obige Bedeutung hat,

im Molverhältnis 2 : 1 umsetzt.

2. Verfahren nach Anspruch 1, dadurch gekennzeichnet, daß man D-Panthenol mit einer Verbindung der Formeln III—V umsetzt.

3. Verfahren nach Anspruch 1 oder 2, dadurch gekennzeichnet, daß man D-Panthenol mit einer Verbindung der Formeln III—V, worin R $C_1-C_{12}$-Alkyl bedeutet, umsetzt.

4. Verfahren nach einem der Ansprüche 1 bis 3, dadurch gekennzeichnet, daß man D-Panthenol mit einer Verbindung der Formeln III oder IV umsetzt, worin R die Isobutylgruppe darstellt.

5. Verfahren nach einem der Ansprüche 1 bis 3, dadurch gekennzeichnet, daß man D-Panthenol mit einer Verbindung der Formel V umsetzt, worin R die Äthylgruppe darstellt.

6. Verfahren nach Anspruch 1 oder 2, dadurch gekennzeichnet, daß man D-Panthenol mit einer Verbindung der Formeln III—V umsetzt, worin R die Isopropyloxygruppe darstellt.

**Claims for the Contracting States: BE, CH, DE, FR, GB, IT, LI, LU, NL, SE**

1. Compound of the general formula

D or DL

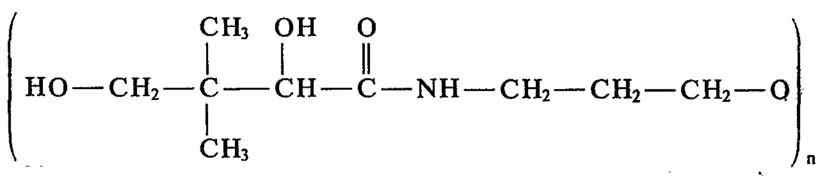 (I)

wherein Me signifies aluminium and n signifies the number 3 or Me signifies zinc and n signifies the number 2.

2. Aluminium D-panthenolate.

3. Zinc D-panthenolate.

4. A compound in accordance with claim 1, 2 or 3 as a topical medicament.

5. A compound in accordance with claim 1, 2 or 3 as a topical wound healing medicament.

6. A process for the manufacture of compounds of general formula I in accordance with claim 1, characterized by reacting D- or DL-panthenol of the formula

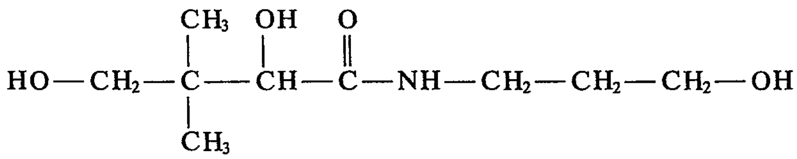 (II)

with a compound of the general formulae

$(R)_3Al$ (III) or $(R)_2AlH$ (IV)

wherein R represents $C_1-C_{12}$-alkyl or $C_1-C_{12}$-alkoxy groups,

in the molar ratio 3 : 1 or with a compound of the general formula

$(R)_2Zn$ (V)

wherein R has the above significance,

in the molar ratio 2 : 1.

7. A topical medicament containing a compound of general formula I in accordance with claim 1.

8. A topical wound healing medicament containing a compound of formula I in accordance with claim 1.

**Claims for the Contracting State AT**

1. A process for the manufacture of compounds of the general formula

D or DL

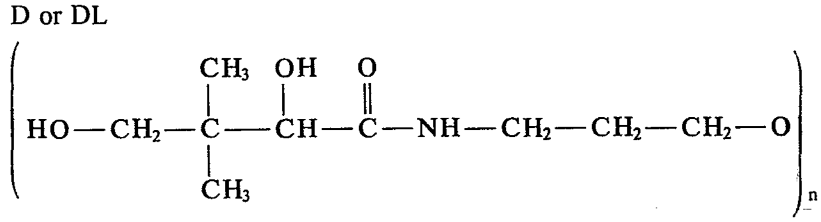 (I)

wherein Me signifies aluminium and n signifies the number 3 or Me signifies zinc and n signifies the number 2,

characterized by reacting D- or DL-panthenol of the formula

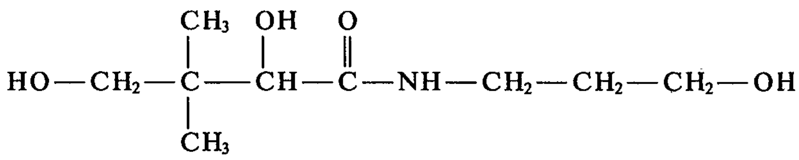

(II)

with a compound of the general formulae

(R)$_3$Al (III) or (R)$_2$AlH (IV)

wherein R represents $C_1-C_{12}$-alkyl or $C_1-C_{12}$-alkoxy groups,

in the molar ratio 3 : 1 or with a compound of the general formula

(R)$_2$Zn (V)

wherein R has the above significance,

in the molar ratio 2 : 1.

2. A process according to claim 1, characterized in that D-panthenol is reacted with a compound of formulae III—V.

3. A process according to claim 1 or 2, characterized in that D-panthenol is reacted with a compound of formulae III—V in which R signifies $C_1-C_{12}$-alkyl.

4. A process according to any one of claims 1 to 3, characterized in that D-panthenol is reacted with a compound of formulae III or IV in which R represents the isobutyl group.

5. A process according to any one of claims 1 to 3, characterized in that D-panthenol is reacted with a compound of formula V in which R represents the ethyl group.

6. A process according to claim 1 or 2, characterized in that D-panthenol is reacted with a compound of formulae III—V in which R represents the isopropyloxy group.

**Revendications pour les états contractants: BE, CH, DE, FR, GB, IT, LI, LU, NL, SE**

1. Composés de formule générale

D ou DL

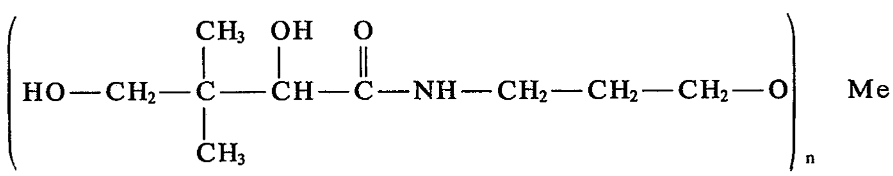

(I)

où Me représente l'aluminium et n le nombre 3, ou bien où Me représente le zinc et n représente le nombre 2.

2. D-panthénolate d'aluminium.

3. D-panthénolate de zinc.

4. Composé selon les revendications 1, 2 et 3 comme médicament à usage local.

5. Composé selon les revendications 1, 2 et 3 comme médicament cicatrisant à usage local.

6. Procédé de préparation de composés de formule générale I selon la revendication 1, caractérisé en ce qu'on fait réagir du D- ou DL-panthénol de formule

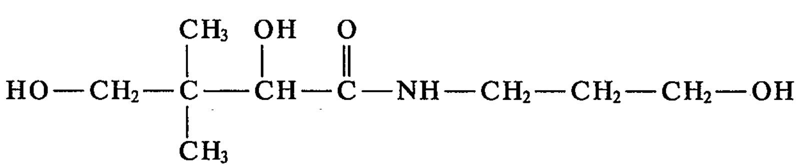

(II)

avec un composé de formules générales

(R)$_3$Al (III) ou (R)$_2$AlH (IV)

où R représente des groupes alcoyle en C1 à C12 ou alcoxy en C1 à C12,
dans un rapport molaire 3 : 1 ou avec un composé de formule générale

$(R)_2Zn$ (V)

où R a la signification donnée ci-dessus, dans un rapport molaire 2 : 1.

7. Médicament à usage local contenant un composé de formule générale I selon la revendication 1.

8. Agent cicatrisant à usage local contenant un composé de formule I selon la revendication 1.

**Revendications pour l'état contractant: AT**

1. Procédé de préparation de composés de formule générale

D ou DL

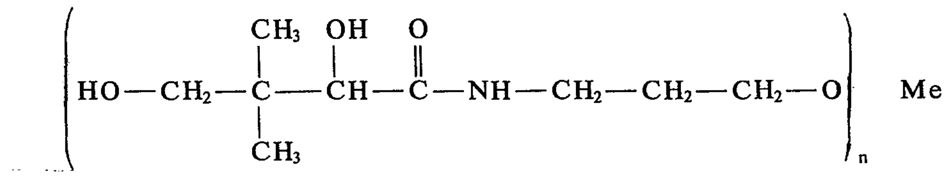

(I)

où Me représente l'aluminium et n le nombre 3, ou bien où Me représente le zinc et n le nombre 2, caractérisé en ce qu'on fait réagir du D- ou DL-panthénol de formule

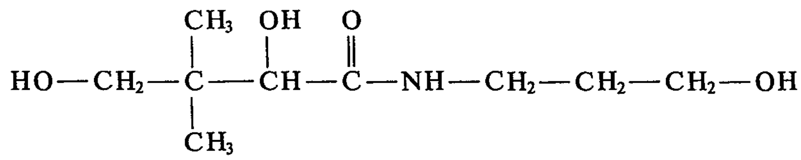

(II)

avec un composé de formules générales

$(R)_3Al$ (III) ou $(R)_2AlH$ (IV)

où R représente des groupes alcoyle en C1 à C12 ou alcoxy en C1 à C12, dans un rapport molaire 3 : 1 ou avec un composé de formule générale

$(R)_2Zn$ (V)

où R a la signification donnée ci-dessus, dans un rapport molaire 2 : 1.

2. Procédé selon la revendication 1, caractérisé en ce qu'on fait réagir du D-panthénol avec un composé de formules III—V.

3. Procédé selon les revendications 1 et 2, caractérisé en ce qu'on fait réagir du D-panthénol avec un composé de formules III—V où R représente un alcoyle en C1 à C12.

4. Procédé selon l'une des revendications 1 à 3, caractérisé en ce qu'on fait réagir du D-panthénol avec un composé de formules III ou IV où R représente le groupe isobutyle.

5. Procédé selon l'une des revendications 1 à 3, caractérisé en ce qu'on fait réagir du D-panthénol avec un composé de formule V où R représente le groupe éthyle.

6. Procédé selon les revendications 1 et 2, caractérisé en ce qu'on fait réagir du D-panthénol avec un composé de formules III—V où R représente le groupe isopropyloxy.